# EUROPEAN PATENT APPLICATION

(11) **EP 4 273 240 A1**
(43) Date of publication of application: **08.11.2023**
(21) Application number: 21915323.6
(22) Date of filing: 28.12.2021
(51) Int. Cl.: C12N 15/09, C07K 14/195

(54) **GENE FUNCTION CONTROL BY NOVEL GUIDE NUCLEIC ACID MECHANISM**

(30) Priority: 29.12.2020 JP 2020219833
(71) Applicant: National University Corporation Kobe University, Kobe-shi, Hyogo 657-8501 (JP)
(72) Inventor: NISHIDA, Keiji, Kobe-shi, Hyogo 657-8501 (JP); MITSUNOBU, Hitoshi, Kobe-shi, Hyogo 657-8501 (JP)
(74) Representative: Weickmann & Weickmann PartmbB
(86) International application number: PCT/JP2021/048908
(87) International publication number: WO 2022/145465

(57) **Abstract**

The present disclosure provides a site-specific modification method of a DNA molecule that enables gene function manipulation.

According to the present disclosure, provided is a method for manipulating gene function by a site-specific action on a target DNA molecule, said method comprising a step for preparing a complex containing an Argonaute protein, a guide RNA and an additional sequence and a step for contacting the target DNA molecule with the complex, wherein the guide RNA is designed to guide the complex to a region containing the desired site in the target DNA molecule.

## Description

### [Technical Field]

The present disclosure relates to a site-specific alteration method for a DNA molecule which enables manipulation of a gene function.

### [Background Art]

In recent years, the CRISPR system of bacteria and archaea has drawn attention as a DNA sequence recognition mechanism by a guide nucleic acid and is applied as a genome editing technique. The CRISPR system utilizes an RNA (guide RNA) of a small molecule having complementarity with a DNA sequence entering a bacterium to promote targeting to and decomposition of an exogenous DNA that is a target.

### [Summary of Invention]

### [Solution to Problem]

On the other hand, it is known that there is an Argonaute protein derived from some bacteria which is capable of recognizing a DNA sequence by using a guide RNA. This Argonaute protein is a guide RNA-dependent RNA target molecule which is responsible for RNAi in a eukaryote. The present disclosure provides a site-specific alteration method for a DNA molecule which utilizes said Argonaute protein as a novel genome editing mechanism and enables manipulation of a gene function.

Thus, the present disclosure provides the following items.

### (Item 1)

A method for manipulating a gene function by a site-specific action on a target DNA molecule, the method comprising:
providing a complex comprising an Argonaute protein, a guide RNA, and an added sequence; and
contacting the complex with the target DNA molecule,
wherein the guide RNA is designed to guide the complex to a region comprising a site of interest in the target DNA molecule.

### (Item 2)

The method of the preceding item, wherein the Argonaute protein has an activity of targeting a DNA.

### (Item 3)

The method of any one of the preceding items, wherein the Argonaute protein has an activity of targeting a DNA at a physiological temperature.

### (Item 4)

The method of any one of the preceding items, wherein the Argonaute protein is derived from a prokaryote.

### (Item 5)

The method of any one of the preceding items, wherein the Argonaute protein is derived from a bacterium.

### (Item 6)

The method of any one of the preceding items, wherein the Argonaute protein is RsAgo derived from *Rhodobacter.*

### (Item 7)

The method of any one of the preceding items, wherein the complex is formed outside of a living body.

### (Item 8)

The method of any one of the preceding items, wherein the complex is formed outside of a living body, and the target DNA molecule is present in a living body.

### (Item 9)

The method of any one of the preceding items, wherein the Argonaute-guide RNA complex is designed to be directed to a target DNA molecule having a target proximate motif sequence.

### (Item 10)

The method of any one of the preceding items, wherein manipulation of the gene function includes gene expression control, gene sequence alteration, and/or both thereof.

### (Item 11)

The method of any one of the preceding items, wherein manipulation of the gene function includes gene expression control.

### (Item 12)

The method of any one of the preceding items, wherein manipulation of the gene function includes gene sequence alteration.

### (Item A1)

A method for regulating expression of a target DNA molecule, the method comprising:
providing a complex comprising an Argonaute protein, a guide RNA, and an added sequence;
dissociating at least a part of a double stranded DNA sequence in the target DNA molecule; and
contacting the complex with the double stranded DNA sequence at least a part of which has been dissociated,
wherein the guide RNA is designed to guide the complex to a region comprising the double stranded DNA sequence at least a part of which has been dissociated.

### (Item A2)

The method of any one of the preceding items, wherein the Argonaute protein has an activity of targeting a DNA.

### (Item A3)

The method of any one of the preceding items, wherein the Argonaute protein has an activity of targeting a DNA at a physiological temperature.

### (Item A4)

The method of any one of the preceding items, wherein the Argonaute protein is derived from a prokaryote.

### (Item A5)

The method of any one of the preceding items, wherein the Argonaute protein is derived from a bacterium.

### (Item A6)

The method of any one of the preceding items, wherein the Argonaute protein is RsAgo derived from *Rhodobacter.*

### (Item A7)

The method of any one of the preceding items, wherein the complex is formed outside of a living body.

### (Item A8)

The method of any one of the preceding items, wherein the complex is formed outside of a living body, and the target DNA molecule is present in a living body.

### (Item B1)

An Argonaute-guide RNA complex, comprising:
an Argonaute protein; and
a synthesized guide RNA polynucleotide,
wherein the guide sequence is designed so that the Argonaute-guide RNA complex is directed to a target DNA molecule having a target proximate motif sequence.

### (Item B2)

The complex of any one of the preceding items, wherein the Argonaute protein has an activity of targeting a DNA.

### (Item B3)

The complex of any one of the preceding items, wherein the Argonaute protein has an activity of targeting a DNA at a physiological temperature.

### (Item B4)

The complex of any one of the preceding items, wherein the Argonaute protein is derived from a prokaryote.

### (Item B5)

The complex of any one of the preceding items, wherein the Argonaute protein is derived from a bacterium.

### (Item B6)

The complex of any one of the preceding items, wherein the Argonaute protein is RsAgo derived from *Rhodobacter.*

### (Item C1)

An Argonaute-guide RNA complex for use in manipulating a gene function by a site-specific action on a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to site-specifically act on the target DNA molecule, thereby manipulating the gene function.

### (Item C2)

The complex of any one of the preceding items, wherein the Argonaute protein has an activity of targeting a DNA.

### (Item C3)

The complex of any one of the preceding items, wherein the Argonaute protein has an activity of targeting a DNA at a physiological temperature.

### (Item C4)

The complex of any one of the preceding items, wherein the Argonaute protein is derived from a prokaryote.

### (Item C5)

The complex of any one of the preceding items, wherein the Argonaute protein is derived from a bacterium.

### (Item C6)

The complex of any one of the preceding items, wherein the Argonaute protein is RsAgo derived from *Rhodobacter.*

### (Item C7)

The complex of any one of the preceding items, wherein manipulation of the gene function includes gene expression control, gene sequence alteration, and/or both thereof.

### (Item C8)

The complex of any one of the preceding items, wherein manipulation of the gene function includes gene expression control.

### (Item C9)

The complex of any one of the preceding items, wherein manipulation of the gene function includes gene sequence alteration.

### (Item D1)

An Argonaute-guide RNA complex for use in regulating expression of a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to regulate expression of the target DNA molecule.

### (Item D2)

The complex of any one of the preceding items, wherein the Argonaute protein has an activity of targeting a DNA.

### (Item D3)

The complex of any one of the preceding items, wherein the Argonaute protein has an activity of targeting a DNA at a physiological temperature.

### (Item D4)

The complex of any one of the preceding items, wherein the Argonaute protein is derived from a prokaryote.

### (Item D5)

The complex of any one of the preceding items, wherein the Argonaute protein is derived from a bacterium.

### (Item D6)

The complex of any one of the preceding items, wherein the Argonaute protein is RsAgo derived from *Rhodobacter.*

### (Item E1)

A system or kit for manipulating a gene function by a site-specific action on a target DNA molecule, the system or kit comprising:
two or more complexes comprising an Argonaute protein, a guide RNA, and an added sequence, or two or more thereof; and
a means or reagent for contacting the complexes with the target DNA molecule,
wherein the guide RNA is designed to guide the complexes to a region comprising a site of interest in the target DNA molecule.

### (Item E2)

The system or kit of any one of the preceding items, wherein the Argonaute protein has an activity of targeting a DNA.

### (Item E3)

The system or kit of any one of the preceding items, wherein the Argonaute protein has an activity of targeting a DNA at a physiological temperature.

### (Item E4)

The system or kit of any one of the preceding items, wherein the Argonaute protein is derived from a prokaryote.

### (Item E5)

The system or kit of any one of the preceding items, wherein the Argonaute protein is derived from a bacterium.

### (Item E6)

The system or kit of any one of the preceding items, wherein the Argonaute protein is RsAgo derived from *Rhodobacter.*

### (Item E7)

The system or kit of any one of the preceding items, comprising a means for forming the complexes outside of a living body.

### (Item E8)

The system or kit of any one of the preceding items, comprising a means for forming the complexes outside of a living body, wherein the target DNA molecule is present in a living body.

### (Item E9)

The system or kit of any one of the preceding items, wherein the Argonaute-guide RNA complex is designed to be directed to a target DNA molecule having a target proximate motif sequence.

### (Item E10)

The system or kit of any one of the preceding items, wherein manipulation of the gene function includes gene expression control, gene sequence alteration, and/or both thereof.

### (Item E11)

The system or kit of any one of the preceding items, wherein manipulation of the gene function includes gene expression control.

### (Item E12)

The system or kit of any one of the preceding items, wherein manipulation of the gene function includes gene sequence alteration.

### (Item F1)

A system or kit for regulating expression of a target DNA molecule, the system or kit comprising:
two or more complexes comprising an Argonaute protein, a guide RNA, and an added sequence, or two or more thereof;
a means or reagent for dissociating at least a part of a double stranded DNA sequence in the target DNA molecule; and
a means or reagent for contacting the complexes with the double stranded DNA sequence at least a part of which has been dissociated,
wherein the guide RNA is designed to guide the complexes to a region comprising the double stranded DNA sequence at least a part of which has been dissociated.

### (Item F2)

The system or kit of any one of the preceding items, wherein the Argonaute protein has an activity of targeting a DNA.

### (Item F3)

The system or kit of any one of the preceding items, wherein the Argonaute protein has an activity of targeting a DNA at a physiological temperature.

### (Item F4)

The system or kit of any one of the preceding items, wherein the Argonaute protein is derived from a prokaryote.

### (Item F5)

The system or kit of any one of the preceding items, wherein the Argonaute protein is derived from a bacterium.

### (Item F6)

The system or kit of any one of the preceding items, wherein the Argonaute protein is RsAgo derived from *Rhodobacter.*

### (Item F7)

The system or kit of any one of the preceding items, further comprising a means for forming the complexes outside of a living body.

### (Item F8)

The system or kit of any one of the preceding items, further comprising a means for forming the complexes outside of a living body, wherein the target DNA molecule is present in a living body.

### (Item G1)

Use of an Argonaute-guide RNA complex for use in manipulating a gene function by a site-specific action on a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to site-specifically act on the target DNA molecule, thereby manipulating the gene function.

### (Item G2)

The use of any one of the preceding items, wherein the Argonaute protein has an activity of targeting a DNA.

### (Item G3)

The use of any one of the preceding items, wherein the Argonaute protein has an activity of targeting a DNA at a physiological temperature.

### (Item G4)

The use of any one of the preceding items, wherein the Argonaute protein is derived from a prokaryote.

### (Item G5)

The use of any one of the preceding items, wherein the Argonaute protein is derived from a bacterium.

### (Item G6)

The use of any one of the preceding items, wherein the Argonaute protein is RsAgo derived from *Rhodobacter.*

### (Item G7)

The use of any one of the preceding items, wherein manipulation of the gene function includes gene expression control, gene sequence alteration, and/or both thereof.

### (Item G8)

The use of any one of the preceding items, wherein manipulation of the gene function includes gene expression control.

### (Item G9)

The use of any one of the preceding items, wherein manipulation of the gene function includes gene sequence alteration.

### (Item H1)

Use of an Argonaute-guide RNA complex for use in regulating expression of a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to regulate expression of the target DNA molecule.

### (Item H2)

The use of any one of the preceding items, wherein the Argonaute protein has an activity of targeting a DNA.

### (Item H3)

The use of any one of the preceding items, wherein the Argonaute protein has an activity of targeting a DNA at a physiological temperature.

### (Item H4)

The use of any one of the preceding items, wherein the Argonaute protein is derived from a prokaryote.

### (Item H5)

The use of any one of the preceding items, wherein the Argonaute protein is derived from a bacterium.

### (Item H6)

The use of any one of the preceding items, wherein the Argonaute protein is RsAgo derived from *Rhodobacter.*

The present disclosure is intended so that one or more of the above features can be provided not only as the explicitly disclosed combinations, but also as other combinations thereof. Additional embodiments and advantages of the present disclosure are recognized by those skilled in the art by reading and understanding the following detailed explanation, as needed.

Features and significant actions/effects of the present disclosure other than those described above will be clear to those skilled in the art by referring to the section of the embodiments below and the drawings.

### [Advantageous Effects of Invention]

The method of the present disclosure can provide a new gene alteration technique with a smaller molecule size and less restriction on a target sequence as compared to the conventional CRISPR system. Further, the Argonaute protein-guide RNA complex of the present disclosure can target-specifically regulate gene expression and is also capable of localizing an effector which acts on a DNA via an aptamer or the like added to the guide RNA. Thus, said complex can be expected to be applied to various types of genome editing.

### [Brief Description of Drawings]

[Figure 1] Figure **1** is a schematic diagram showing fusion of an aptamer with the Argonaute protein-guide RNA complex of the present disclosure.
[Figure 2] Figure **2** is a schematic diagram showing purification of a recombinant RsAgo protein.
[Figure 3] Figure **3** is a schematic diagram showing the test confirming the nucleic acid binding capability.
[Figure 4] Figure **4** shows the test result of the nucleic acid binding capability of an RsAgo protein.
[Figure 5] Figure **5** shows the test result of the binding affinity of an RsAgo protein to a target sequence.
[Figure 6] Figure **6** shows the test result of the sequence dependency of binding of an RsAgo protein to a target sequence.
[Figure 7] Figure **7** is a schematic diagram showing synthesis of a guide RNA-aptamer by in vitro transcription.
[Figure 8] Figure **8** is a diagram showing the result of synthesis of a guide RNA added with an aptamer.
[Figure 9] Figure **9** is a diagram showing the result of binding of the guide RNA-aptamer to a target sequence.
[Figure 10] Figure **10** shows the result showing sequence-specific DNA binding of the RNA added with RsAgo and an aptamer.
[Figure 11] Figure **11** is a diagram showing the result of binding of an RsAgo protein to a partial single stranded region.
[Figure 12] Figure **12** shows the result showing a specific binding property of an RsAgo protein to a single stranded DNA comprising a target sequence.
[Figure 13] Figure **13** is a diagram showing the result of the action of an RsAgo protein on a gene expression region.

### [Description of Embodiments]

The present disclosure is described hereinafter while showing the best modes thereof. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The definitions of the terms and/or basic technical contents that are particularly used herein are described hereinafter when appropriate.

As used herein, "about" refers to a range of ± 10% from the numerical value that is described subsequent to "about".

As used herein, "Argonaute protein" (Ago) is a protein constituting an RISC (RNA-induced silencing complex) that is a central structure in RNA silencing via a small molecule RNA. The protein binds to a single stranded RNA which functions as a guide to form an RISC, and this complex catalyzes cleavage of an mRNA of a sequence complementary to the single stranded RNA bound to the Argonaute. An Argonaute protein is a modulator essential to gene expression in a plant or an animal. An Ago consists of four domains (N domain, PAZ domain, MID domain, and PIWI domain) and two linkers (L1 linker and L2 linker) and has a molecular weight of about 100 kDa. A small RNA such as siRNA or miRNA is generally biosynthesized as a double stranded RNA and binds to an Ago as a double strand. One of the double stranded RNA strands (passenger strand) is torn off in the Ago, whereby a mature RISC in which only the other strand (guide strand) is incorporated into the Ago is formed. The RISC formed in this manner utilizes the sequence complementarity of the guide strand to find out a specific target mRNA, and causes suppression of translation and destabilization due to a shortened poly A strand (miRNA pathway). In addition, proteins that belong to the Ago family include those with an endonuclease activity called "slicer". Such Agos are also able to cleave a target mRNA (RNAi pathway). Both Agos are included herein. Examples of a representative Ago can include Entry PS50822 of Prosite (https://prosite.expasy.org/PS50822, and *Rhodobacter sphaeroides* ATCC 17025 (GenBank: ABP72561.1, https://www.uniprot.org/uniprot/Q9UKV8) can be preferably used.

As used herein, "guide RNA" refers to an RNA molecule comprising: a sequence complementary to a target; and a motif sequence required for forming a complex with an effector protein or a structure for the same. A guide RNA is an RNA that leads an Argonaute protein to specifically target a DNA sequence and has a function of binding to the Argonaute protein of the present disclosure to lead the protein to a target DNA.

As used herein, "target proximate motif sequence" refers to a sequence which is present in a target double stranded polynucleotide and can be recognized by an Argonaute protein and/or Argonaute protein-guide RNA complex. The length and the base sequence of a target proximate motif sequence vary depending on the bacterium species from which the Argonaute protein is derived. For example, for RsAgo derived from *Rhodobacter sphaeroides,* the target only needs to have only T on the 5' side as a target proximate motif sequence.

As used herein, "endonuclease" refers to an enzyme that cleaves a nucleotide strand in the middle thereof. For example, when the Argonaute protein of the present disclosure has an endonuclease activity, the Argonaute protein has an enzymatic activity of being guided by a guide RNA and cleaving a DNA strand.

As used herein, "manipulation of a gene function" includes cleavage, alteration, and/or modification of a DNA sequence as well as suppression, control, and/or regulation of expression of a gene consisting of the DNA sequence.

As used herein, "protein" refers to a polymer of amino acid residues and is interchangeably used with "polypeptide" or "peptide". Further, the term refers to an amino acid polymer in which one or more amino acids are chemical analogs or modified derivatives of the corresponding naturally-occurring amino acids.

As used herein, "DNA sequence" refers to a nucleotide sequence with any length, which is a deoxyribonucleotide or a ribonucleotide, may be linear, cyclic, or branched, and is a single strand or a double strand.

Furthermore, as used herein, "N" refers to any one base selected from the group consisting of adenine, cytosine, thymine, and guanine. "A" refers to adenine, "G" refers to guanine, "C" refers to cytosine, "T" refers to thymine, "R" refers to a base having a purine backbone (adenine or guanine), and "Y" refers to a base having a pyrimidine backbone (cytosine or thymine).

As used herein, "polynucleotide" refers to a deoxyribonucleotide or ribonucleotide polymer which is in linear or cyclic conformation and in either a single stranded or double stranded form. A "polynucleotide" should not be construed as limiting with respect to the length of a polymer. Further, said term encompasses a known analog of a natural nucleotide as well as a nucleotide which is modified in at least one of the base moiety, the sugar moiety, and the phosphate moiety (e.g., phosphorothioate backbone). In general, an analog of a specific nucleotide has the same base-pairing specificity. For example, an analog of A base-pairs with T.

As used herein, "added sequence" refers to any sequence that can be added to a guide RNA, which is a sequence for enabling the guide RNA to specifically bind to a specific molecule and/or imparting a catalytic activity. Examples of an added sequence include any nucleic acid aptamer.

### (Preferred embodiments)

Preferred embodiments of the present disclosure are described below. Embodiments provided below are provided to facilitate the understanding of the present disclosure. The scope of the present disclosure should not be limited to the following descriptions. Thus, it is apparent that those skilled in the art can make appropriate modifications within the scope of the present disclosure by referring to the descriptions herein. Further, the following embodiments of the present disclosure can be used alone or in combination.

One aspect of the present disclosure provides a method for manipulating a gene function by a site-specific action on a target DNA molecule, the method comprising: providing a complex comprising an Argonaute protein, a guide RNA, and an added sequence; and contacting the complex with the target DNA molecule, wherein the guide RNA is designed to guide the complex to a region comprising a site of interest in the target DNA molecule. The method of the present disclosure is useful as a genome editing technique.

While the CRISPR system is mainly used for a conventional genome editing technique, there is a locus for which target designing is impossible because the CRISPR system has a restriction in that an RNA molecule of about 20 bases located in the front and back of a PAM sequence of about 2 bases to 5 bases specifying target specificity is utilized for target targeting. Further, when there is a similar sequence near a sequence of interest to be cleaved, the similar sequence may also be cleaved. Furthermore, among genome editing techniques using the CRISPR system, those used in practical applications have a relatively large molecular weight, in which problems remain in terms of delivery into a cell and immunogenicity.

Thus, development of a new targeting system and a new RNA-guided endonuclease which do not have these problems is desired. Some Argonaute proteins derived from a bacterium are able to recognize a DNA sequence by using a guide RNA. In one embodiment of the present disclosure, said Argonaute proteins derived from a bacterium can be utilized as a novel genome editing mechanism.

In one embodiment, an Argonaute protein (Ago) used in the present disclosure may be derived from any organism as long as the Argonaute protein comprises an RNA-guided type DNA targeting activity. An Argonaute protein may also be derived from a prokaryote. An Argonaute protein is preferably derived from a bacterium. For example, MpAgo derived from *Marinitoga piezophila,* TpAgo derived from *Thermotoga profunda,* RsAgo derived from *Rhodobacter sphaeroides* or the like can be used as an Argonaute protein. In another embodiment, an Argonaute protein derived from *Mesorhizobium sp, Bradyrhizobium centrolobii, Calothrix desertica* PCC 7102, *Planctomycetes* bacterium Mal33, *Elusimicrobia* bacterium RBG_16_66_12, *Dehalococcoides mccartyi* or the like can be used as an Argonaute protein used in the present disclosure. Further, in another embodiment, a family including homologs of these Argonaute proteins can also be used.

An Argonaute protein has a molecular weight of about 100 kDa, which can be greatly reduced as compared to 160 kDa of CRISPR-Cas9 of *Streptococcus pyogenes,* which is widely used in a conventional genome editing technique. In addition, a guide RNA which binds to an Argonaute protein is also smaller because said guide RNA does not have a Scaffold structure sequence or the like. A sequence that can be targeted by the Argonaute protein-guide RNA complex of the present disclosure only needs to have only T on the 5' side as a target proximate motif sequence. As a result, there is only less restriction than GG, which is a PAM sequence required as target recognition in standard CRISPR-Cas9 of *Streptococcus pyogenes.*

In one embodiment of the present disclosure, a complex of an Argonaute protein and a guide RNA can be formed outside of a cell or outside of a living body in order to have the Argonaute protein retain the selectivity of the guide RNA in a cell. In one embodiment, a target DNA molecule is present in a living body, and an Argonaute protein-guide RNA complex formed outside of a cell or outside of a living body binds in a living body. Further, in another embodiment, an added sequence can be further bound to the guide RNA to achieve a shape to which an effector such as an enzyme site to act on the target DNA can be bound. An added sequence can be any sequence as long as the sequence can specifically bind to a specific molecule. Examples thereof can include an aptamer, a ribozyme, a sequence or structure recognized by an RNA-binding protein and the like. Further, in one embodiment of the present disclosure, MS2 aptamer, PP7, TAR or the like can be used as an aptamer.

In one embodiment of the present disclosure, a complex of an Argonaute protein and an aptamer-fused guide RNA can specifically bind to a target DNA sequence. In one embodiment, an Argonaute protein can have an activity of targeting a DNA, and preferably an activity of targeting a DNA at a physiological temperature.

Further, in another embodiment, the complex of an Argonaute protein and a guide RNA of the present disclosure can regulate expression of a gene by performing targeting while inducing the gene expression in a closed double stranded DNA. For example, expression of a target gene can be improved or activated by binding a transcription factor to the complex of an Argonaute protein and a guide RNA of the present disclosure via a fusion protein, an aptamer or the like. Preferably, the Argonaute-guide RNA complex of the present disclosure can specifically bind to a double stranded DNA in which gene expression has been induced and suppress the gene expression. For example, expression of a target gene can be suppressed by designing a guide RNA so that it binds to the strand on the opposite side to the template of transcription of the target gene.

In one embodiment of the present disclosure, a guide RNA only needs to be an RNA which leads an Argonaute protein to specifically target a DNA sequence. A guide RNA is preferably designed to guide an Argonaute-guide RNA complex to a region comprising a site of interest in a target DNA molecule. A guide RNA recognizes a target DNA having only T on the 5' side as a target proximate motif sequence and binds to the complementary sequence thereof. In one embodiment, a guide RNA has a sequence complementary to a target DNA at the 5' terminal of the guide RNA and binds to the target DNA via the complementary sequence, thereby leading the Argonaute protein of the present disclosure to the target DNA. In one embodiment, when an Argonaute protein functions as a DNA endonuclease, it is possible to cleave DNA at a site in which a target DNA is present and, for example, specifically eliminate the function of the target DNA. A guide RNA is designed and prepared based on sequence information of a target DNA to be cleaved or modified. Specific examples include the sequences that are used in the Examples.

In one embodiment of the present disclosure, the Argonaute protein-guide RNA complex of the present disclosure can be provided as a complex comprising an Argonaute protein, a guide RNA, and an added sequence. For example, in one embodiment, an Argonaute protein and a DNA or mRNA for expressing said protein; a guide RNA comprising any target sequence with a 5' terminal starting with phosphorylated U; and any sequence such as an aptamer to be added on the 3' side of the guide RNA can be prepared to form a complex outside of a cell or in a cell.

In one embodiment of the present disclosure, contacting an Argonaute protein-guide RNA complex with a target DNA molecule comprises binding the Argonaute protein-guide RNA complex of the present disclosure to a DNA comprising a target sequence by, for example, making the double stranded DNA partially dissociated through gene expression or the like. In one embodiment of the present disclosure, the Argonaute protein-guide RNA complex of the present disclosure binds to the DNA comprising a target sequence upon gene expression in this manner, whereby the gene expression can be controlled.

Another aspect of the present disclosure provides a method for regulating expression of a target DNA molecule, the method comprising: providing a complex comprising an Argonaute protein, a guide RNA, and an added sequence; dissociating at least a part of a double stranded DNA sequence in the target DNA molecule; and contacting the complex with the double stranded DNA sequence at least a part of which has been dissociated, wherein the guide RNA is designed to guide the complex to a region comprising the double stranded DNA sequence at least a part of which has been dissociated.

In one embodiment of the present disclosure, dissociating at least a part of a double stranded DNA sequence in a target DNA molecule comprises, for example, making the double stranded DNA partially dissociated through gene expression or the like.

In one embodiment of the present disclosure, contacting an Argonaute protein-guide RNA complex with a double stranded DNA sequence at least a part of which has been dissociated comprises binding the Argonaute protein-guide RNA complex of the present disclosure to the double stranded DNA sequence at least a part of which has been dissociated by, for example, making the double stranded DNA partially dissociated through gene expression or the like. In one embodiment of the present disclosure, the Argonaute protein-guide RNA complex of the present disclosure binds to the DNA comprising a target sequence upon gene expression in this manner, whereby the gene expression can be controlled.

In one embodiment of the present disclosure, the complex of an Argonaute protein and a guide RNA of the present disclosure can be obtained by various methods, but the complex can be preferably obtained by the method described in the present Examples. In another embodiment, the complex of an Argonaute protein and a guide RNA of the present disclosure can be obtained by any method as long as the complex exerts the action or effect shown herein.

In one embodiment of the present disclosure, the Argonaute protein-guide RNA complex of the present disclosure can target a site at which a double stranded structure is dissociated by gene expression or DNA replication in a target cell and suppressively control expression of a gene comprising the target site, thereby changing the cell function or analyzing the gene function. Further, in another embodiment, in order to target only a specific cell group in heterogeneous cell populations, the Argonaute protein-guide RNA complex of the present disclosure can control a gene function by targeting a sequence which only some cell groups of the population possess.

Further, in another embodiment, the Argonaute protein-guide RNA complex of the present disclosure is also able to cause an increase in target site-specific gene expression by being bound to a transcription control factor via an aptamer or the like.

As a genome editing application, it is possible to alter a target sequence and surrounding DNAs by binding a nuclease, a recombinase, a deaminase, or a glycosylase or the like to the Argonaute protein-guide RNA complex of the present disclosure via an aptamer or the like. In the same manner, it is possible to change a target sequence and the state of a surrounding chromatin modification by binding a chromatin modification factor such as a methylation enzyme, demethylation enzyme, or histone acetylation enzyme.

In another aspect, the present disclosure provides a method for altering a target site of a double stranded DNA (e.g., chromosomal DNA, mitochondrial DNA, or chloroplast DNA; hereinafter, these DNAs are also collectively referred to as "genomic DNA"). The method is characterized by comprising contacting a complex of an Argonaute protein and a guide RNA with the double stranded DNA.

In the present disclosure, "alteration" of a double stranded DNA means that a certain nucleotide (e.g., dA, dC, dG, or dT) or nucleotide sequence on a DNA strand is substituted with another nucleotide or nucleotide sequence, or another nucleotide or nucleotide sequence is inserted between certain nucleotides on a DNA strand. In this regard, although the double stranded DNA to be altered is not particularly limited, said double stranded DNA is preferably a genomic DNA.

In the present disclosure, "donor DNA" refers to DNA comprising an exogenous insertion sequence. A donor DNA generally comprises two types of sequences (hereinafter, also referred to as "homology arms") homologous to sequences of two regions at the upstream side and downstream side of a target site, which are adjacent to the target site (hereinafter, also referred to as "adjacent regions"). Each homology arm may be distinguished as the "5' homology arm" and "3' homology arm". A "target site" of a double stranded DNA refers to a region that would be substituted with an insertion sequence contained in the donor DNA, or a site between nucleotides where the insertion sequence would be inserted. The target site does not include the aforementioned adjacent sequences.

A sequence homologous to an adjacent region of a target site may be not only a completely identical sequence, but also a sequence with identity of preferably 80% or greater (e.g., 85% or greater, 90% or greater, 95% or greater, 96% or greater, 97% or greater, 98% or greater, or 99% or greater) to the completely identical sequence, as long as homologous recombination can occur within a cell.

A donor DNA may be a linear (e.g., synthetic double stranded DNA) or cyclic (e.g., plasmid DNA), single stranded DNA (e.g., single stranded oligodeoxynucleotide (ssODN)) or double stranded DNA. A donor DNA can be appropriately designed depending on the base length of an insertion sequence, homologous recombination activity of a host cell, or the like. For example, when the base length of an insertion sequence is 100 bases or less, ssODN or synthetic double stranded DNA is generally used. When longer, a synthetic double stranded DNA or plasmid DNA is generally used. The length of a donor DNA is not particularly limited. The length can be appropriately designed depending on the length of an insertion sequence or the like. The length of an insertion sequence is not particularly limited. The length can be appropriately designed in accordance with the objective, generally in the range of one base to 10s of thousands of bases (e.g., 100 bases or less for ssODN (e.g., 70 bases or less or 50 bases or less)). The length of each homology arm is also not particularly limited. When a donor DNA is ssODN, those with a length of 10 bases to 150 bases are generally used. When a donor DNA is a synthetic double stranded DNA, those with a length of 10 to 5000 bases are generally used. When a donor DNA is a plasmid DNA, those with a length of generally 100 bases to 5000 bases and preferably 500 bases to 1000 bases are used. Such donor DNAs can be designed by referring to a known document (e.g., Ochiai H, Int J Mol Sci, 16:21128-21137 (2015), Hockemeyer D et al., Nat Biotechnol, 27:851-857 (2009)).

In the present disclosure, "nucleic acid sequence recognition module" refers to a molecule or molecule complex having the ability to specifically recognize and bind to a specific nucleotide sequence (i.e., target nucleotide sequence) on a DNA strand. A nucleic acid sequence recognition module binds to a target nucleotide sequence to enable an Argonaute protein linked to the module to specifically act on a targeted site (i.e., target nucleotide sequence and nucleotide in the vicinity thereof) of the Argonaute protein or the like of a double stranded DNA.

As shown in the Examples described below, it has been demonstrated that a target site can be altered by introducing: a complex of an Argonaute protein and a nucleic acid sequence recognition module; and a donor DNA into a cell.

In the present disclosure, "nucleic acid altering enzyme complex" refers to a molecule complex consisting of a complex wherein the nucleic acid sequence recognition module described above is linked with an Argonaute protein, having catalyst function for a nucleic acid base conversion reaction or base removal reaction with a specific nucleotide sequence recognizing capability imparted. In this regard, "complex" encompasses not only those comprised of a plurality of molecules, but also those having a nucleic acid sequence recognition module and an Argonaute protein or the like within a single molecule like a fusion protein.

The origin from which an Argonaute protein is derived is not particularly limited. For example, MpAgo derived from *Marinitoga piezophila,* TpAgo derived from *Thermotoga profunda,* RsAgo derived from *Rhodobacter sphaeroides,* and the like can be used. In another embodiment, an Argonaute protein derived from *Mesorhizobium sp, Bradyrhizobium centrolobii, Calothrix desertica* PCC 7102, *Planctomycetes* bacterium Mal33, *Elusimicrobia* bacterium RBG_16_66_12, *Dehalococcoides mccartyi* or the like can be used as an Argonaute protein used in the present disclosure. Further, in another embodiment, a family including homologs of these Argonaute proteins can also be used.

A DNA encoding an Argonaute protein or the like (i.e., DNA encoding an Argonaute protein or DNA encoding a DNA glycosylase) can also be cloned in the same manner by synthesizing an oligo DNA primer based on cDNA sequence information of the enzyme to be used and amplifying it by RT-PCR using a total RNA or mRNA fraction prepared with a cell producing said enzyme as a template. For example, a DNA encoding an Argonaute protein can be cloned by designing a suitable primer for the upstream and the downstream of CDS based on the cDNA sequence (*Rhodobacter sphaeroides* ATCC 17025, https://www.ncbi.nlm.nih.gov/protein/ABP72561.1) registered in the NCBI database and using RT-PCR from an mRNA. Further, a donor DNA can also be cloned in the same manner described above, based on sequence information of a target site or the like.

Alternatively, it is also possible to obtain a sequence optimized for gene expression by artificial synthesis of a gene while taking into consideration the codon frequency, mRNA sequence structure or the like in a targeted host cell.

As an expression vector, an *E. coli* derived plasmid (e.g., pBR322, pBR325, pUC12, or pUC13); *Bacillus subtilis* derived plasmid (e.g., pUB1 10, pTP5, or pC194); yeast derived plasmid (e.g., pSH19 or pSH15); insect cell expressed plasmid (e.g., pFast-Bac); animal cell expressed plasmid (e.g., pA1-11, pXT1, pRc/CMV, pRc/RSV, or pcDNAI/Neo); bacteriophage such as λ phage; insect viral vector such as Baculoviridae viral vector (e.g., BmNPV or AcNPV); animal viral vector such as a retrovirus, vaccinia virus, or adenovirus vector, or the like is used.

A promoter may be any promoter, as long as it is compatible and suitable for a host used in the expression of a gene. Since conventional methods involving DSB may significantly reduce the survival rate of a host cell due to toxicity, it is desirable to increase the cell count until the start of induction by using an induction promoter. Meanwhile, sufficient cell growth is achieved even if the nucleic acid altering enzyme complex of the present disclosure is expressed. Thus, constituent promotors can also be used without restriction.

When a host is, for example, an animal cell, SRα promoter, SV40 promoter, LTR promoter, CMV (cytomegalovirus) promoter, RSV (Rous sarcoma virus) promoter, MoMuLV (Moloney murine leukemia virus) LTR, HSV-TK (herpes simplex virus thymidine kinase) promoter, and the like are used. In particular, CMV promoter, SRα promoter, and the like are preferable.

When a host is *E. coli,* trp promoter, lac promoter, recA promoter, λP_{L} promoter, lpp promoter, T7 promoter, and the like are preferable.

When a host is a *Bacillus* bacteria, SPO1 promoter, SPO2 promoter, penP promoter, and the like are preferable.

When a host is a yeast, Gal1/10 promoter, PHOS promoter, PGK promoter, GAP promoter, ADH promoter, and the like are preferable.

When a host is an insect cell, polyhedrin promoter, P10 promoter, and the like are preferable.

When a host is a plant cell, CaMV35S promoter, CaMV19S promoter, NOS promoter, and the like are preferable.

In addition to those described above, an expression vector comprising an enhancer, splicing signal, terminator, polyadenylation signal, drug resistant gene, auxotrophic complementary gene, other selection markers, replication point, and the like can also be used as desired.

An RNA encoding a nucleic acid sequence recognition module and/or Argonaute protein or the like can be prepared by, for example, transcription to an mRNA in a known in vitro transcription system using a vector encoding the DNA encoding the above-described nucleic acid sequence recognition module and/or Argonaute protein or the like as a template.

A complex of a nucleic acid sequence recognition module and an Argonaute protein or the like can be expressed in a cell by introducing an expression vector comprising the DNA encoding the nucleic acid sequence recognition module and/or Argonaute protein or the like into a host cell and culturing the host cell.

Examples of a host that is used include *Escherichia* bacteria, *Bacillus* bacteria, yeasts, insect cells, insects, animal cells, and the like.

Examples of *Escherichia* bacteria that are used include *Escherichia coli* K12·DH1 [Proc. Natl. Acad. Sci. USA, 60, 160 (1968)], *Escherichia coli* JM103 [Nucleic Acids Research, 9, 309 (1981)], *Escherichia coli* JA221 [Journal of Molecular Biology, 120, 517 (1978)], *Escherichia coli* HB101 [Journal of Molecular Biology, 41, 459 (1969)], *Escherichia coli* C600 [Genetics, 39, 440 (1954)], and the like.

Examples of *Bacillus* bacteria that are used include *Bacillus subtilis* MI114 [Gene, 24, 255 (1983)], *Bacillus subtilis* 207-21 [Journal of Biochemistry, 95, 87 (1984)], and the like.

Examples of yeasts that are used include *Saccharomyces cerevisiae* AH22, AH22R-, NA87-11A, DKD-5D, and 20B-12, *Schizosaccharomyces pombe* NCYC1913 and NCYC2036, *Pichia pastoris* KM71, and the like.

Examples of insect cells that are used when a virus is AcNPV include cabbage armyworm larva derived cell line (*Spodoptera frugiperda* cell; Sf cells), *Trichoplusia ni* midgut derived MG1 cells, *Trichoplusia ni* egg derived High Five^{™} cells, *Mamestra brassicae* derived cells, *Estigmena acrea* derived cells, and the like. Examples of insect cells that are used when a virus is BmNPV include silkworm derived cell lines (*Bombyx mori* N cells; BmN cells) and the like. Examples of the Sf cells that are used include Sf9 cells (ATCC CRL1711), Sf21 cells [In Vivo, 13, 213-217 (1977)], and the like.

Examples of insects that are used include silkworm larvae, vinegar flies, crickets, and the like [Nature, 315, 592 (1985)].

Examples of animal cells that are used include cell lines of monkey COS-7 cells, monkey Vero cells, Chinese hamster ovary (CHO) cells, dhfr gene knockout CHO cells, mouse L cells, mouse AtT-20 cells, mouse myeloma cells, rat GH3 cells, human embryonic kidney-derived cells (e.g., HEK293 cells), human hepatic cancer-derived cells (e.g., HepG2), human FL cells, or the like, iPS cells and ES cells of humans and other mammals and other pluripotent stem cells, and primary culture cells prepared from various tissues. Zebrafish embryos, Xenopus oocytes, or the like can also be used.

Examples of plant cells that are used include suspension cultured cells, calluses, protoplasts, leaf segments, root segments, and the like prepared from various plants (e.g., cereals such as rice, wheat, and corn, commercial crops such as tomato, cucumber, and eggplant, garden plants such as carnation and prairie gentian, experimental plants such as tobacco and *Arabidopsis thaliana,* and the like).

An expression vector can be introduced in accordance with a known method (e.g., lysozyme method, competent method, PEG method, CaCl₂ co-precipitation, electroporation, microinjection, particle gun method, lipofection, agrobacterium method, and the like) depending on the type of host. A donor DNA can also be introduced into cells by the same methods. When introducing an expression vector and a donor DNA as different molecules, the expression vector and donor DNA may be introduced simultaneously or at different timings.

*E. coli* can be transformed in accordance with the method described in, for example, Proc. Natl. Acad. Sci. USA, 69, 2110 (1972), Gene, 17, 107 (1982) or the like.

A vector can be introduced into *Bacillus* bacteria in accordance with the method described in, for example, Molecular & General Genetics, 168, 111 (1979) or the like.

A vector can be introduced into yeast in accordance with the method described in, for example, Methods in Enzymology, 194, 182-187 (1991), Proc. Natl. Acad. Sci. USA, 75, 1929 (1978) or the like.

A vector can be introduced into insect cells and insects in accordance with the method described in, for example, Bio/Technology, 6, 47-55 (1988) or the like.

A vector can be introduced into animal cells in accordance with the method described in, for example, Saibo Kogaku Bessatsu 8 [Cell Engineering Extra Issue 8] Shin Saibo Kogaku Jikken Purotokoru [New Cell Engineering Experimental Protocol, 263-267 (1995) (published by Shujunsha), Virology, 52, 456 (1973).

Cells introduced with a vector and a donor DNA can be cultured in accordance with a known method depending on the type of host.

When, for example, *E. coli* or *Bacillus* bacterium is cultured, a liquid medium is preferable as the medium used in culture. A medium also preferably comprises a carbon source, nitrogen source, inorganic matter, or the like required for the growth of a transformant. In this regard, examples of the carbon source include glucose, dextrin, soluble starch, sucrose, and the like. Examples of the nitrogen source include ammonium salts, nitrates, corn steep liquor, peptone, casein, meat extract, soybean meal, potato extract, and other inorganic and organic matters. Examples of inorganic matters include calcium chloride, sodium dihydrogen phosphate, magnesium chloride, and the like. A yeast extract, vitamin, growth promoting agent, or the like may also be added to a medium. The pH of a medium is preferably about 5 to about 8.

Preferred examples of a medium when *E. coli* is cultured include an M9 medium comprising glucose and casamino acid [Journal of Experiments in Molecular Genetics, 431-433, Cold Spring Harbor Laboratory, New York 1972]. An agent such as 3β-indoleacrylic acid may be optionally added to a medium for efficient functioning of a promoter. *E. coli* is generally cultured at about 15 to about 43°C. Aeration and agitation may also be applied as needed.

*Bacillus* bacteria are generally cultured at about 30 to about 40°C. Aeration and agitation may also be applied as needed.

Examples of a medium when yeast is cultured include Burkholder minimum medium [Proc. Natl. Acad. Sci. USA, 77, 4505 (1980)], SD medium comprising 0.5% casamino acid [Proc. Natl. Acad. Sci. USA, 81, 5330 (1984)], and the like. The pH of a medium is preferably about 5 to about 8. Yeast is generally cultured at about 20°C to about 35°C. Aeration and agitation may also be applied as needed.

Examples of a medium which is used when insect cells or insects are cultured include Grace's Insect Medium [Nature, 195, 788 (1962)] with an appropriate addition of an additive such as inactivated 10% bovine serum, and the like. The pH of a medium is preferably about 6.2 to about 6.4. Insect cells or insects are generally cultured at about 27°C. Aeration and agitation may also be applied as needed.

Examples of a medium which is used when animal cells are cultured include a minimum essential medium (MEM) comprising about 5 to about 20% fetal bovine serum [Science, 122, 501 (1952)], Dulbecco's Modified Eagle Medium (DMEM) [Virology, 8, 396 (1959)], RPMI 1640 medium [The Journal of the American Medical Association, 199, 519 (1967)], 199 medium [Proceeding of the Society for the Biological Medicine, 73, 1 (1950)], and the like. The pH of a medium is preferably about 6 to about 8. The cells are generally cultured at about 30°C to about 40°C. Aeration and agitation may also be applied as needed.

Examples of a medium which is used when plant cells are cultured include an MS medium, LS medium, B5 medium, and the like. The pH of a medium is preferably about 5 to about 8. The cells are generally cultured at about 20°C to about 30°C. Aeration and agitation may also be applied as needed.

A complex of a nucleic acid sequence recognition module and an Argonaute protein or the like, i.e., a nucleic acid altering enzyme complex, can be expressed in a cell in a manner described above.

An RNA encoding a nucleic acid sequence recognition module and/or Argonaute protein or the like can be introduced into a host cell by microinjection, lipofection, or the like. An RNA can be introduced once or repeatedly for multiple times (e.g., 2 to 5 times) at a suitable interval.

The nucleic acid sequence recognition module of the present disclosure using an Argonaute protein is provided as a complex of an Argonaute protein, gRNA-ap, and BP-Effector.

A BP effector protein used in the present disclosure is not particularly limited, as long as the BP effector protein can form a complex with a guide RNA and change the expression and the function of a gene of interest, but is preferably a nuclease, recombinase, deaminase, glycosylase, methylation enzyme, demethylation enzyme, histone acetylation enzyme, or the like.

A DNA encoding a BP effector protein (including a mutant; the same applies hereinafter) can be cloned from a cell producing said enzyme by a method similar to those described above. Said DNA can be obtained by introducing a mutation to convert a certain amino acid into another amino acid.

Alternatively, a DNA encoding a BP effector protein can also be constructed as a DNA with codon usage suitable for expression in a host cell to be used by chemical synthesis or combination with PCR or Gibson assembly by a method similar to those described above for a DNA encoding a nucleic acid sequence recognition module or a DNA encoding an Argonaute protein.

The obtained BP effector protein and/or nucleic acid altering enzyme can be inserted downstream of a promoter of an expression vector similar to those described above depending on the target cell.

The length of a targeting sequence is not particularly limited, as long as the targeting sequence can specifically bind to a target nucleotide sequence. The length is, for example, 15 to 30 nucleotides, and preferably 18 to 25 nucleotides.

A DNA encoding a guide RNA can also be inserted into an expression vector similar to those described above. As a promoter, a promoter wherein the transcription initiation point is T (U for RNA) or a promoter wherein the 5' terminal is U through processing is preferable.

A DNA encoding a guide RNA is a sequence complementary to a target strand of a target nucleotide sequence and can be chemically synthesized by using a DNA/RNA synthesizer after designing an oligo RNA sequence so that the 5' terminal would be phosphorylated U.

A DNA or RNA encoding an Argonaute protein or the like, a guide RNA, or a DNA encoding the same can be introduced into a host cell by a method similar to those described above depending on the host.

The method of the present disclosure can also alter a target site by using a plurality of target nucleotide sequences at different positions. Thus, in one embodiment of the present disclosure, different target nucleotide sequences, a guide RNA specifically binding to each of the target nucleotide sequences, and an Argonaute protein form a nucleic acid altering enzyme complex. In this regard, a common Argonaute protein or the like can be used.

To express the nucleic acid altering enzyme complex of the present disclosure within a host cell, an expression vector comprising a DNA encoding the nucleic acid altering enzyme complex is introduced into a host cell as described above. Meanwhile, in order to efficiently introduce a mutation, it is desirable that expression of a nucleic acid altering enzyme complex is maintained for a certain period or longer at a certain level or higher. From such a viewpoint, the expression vector is certainly incorporated into a host genome, but sustained expression of a nucleic acid altering enzyme complex increases the risk of off-target cleavage. Thus, it is preferable to quickly remove the DNA after alteration of a target site is successfully achieved. Examples of means for removing a DNA incorporated into a host genome include a method of using a Cre-loxP system or FLP-FRT system, method of using transposon, and the like.

Alternatively, efficient editing of a host genome can be materialized while avoiding the risk of off-target cleavage by transiently expressing the nucleic acid altering enzyme complex of the present disclosure within a host cell only during a period required for a nucleic acid reaction to occur at a desired time and for immobilizing an alteration of a target site. Those skilled in the art can appropriately determine a suitable expression induction period based on the culture conditions used or the like. The expression induction period for a nucleic acid encoding the nucleic acid altering enzyme complex of the present disclosure may be extended beyond the above-described "period required... for immobilizing an alteration of a target site" to the extent that it does not cause side effects to a host cell.

Examples of means for transiently expressing the nucleic acid altering enzyme complex of the present disclosure for a desired period at a desired time include a method of preparing a construct (expression vector) comprising a nucleic acid encoding the nucleic acid altering enzyme complex in a form in which the expression period can be controlled, and introducing the construct into a host. Specific examples of "form in which the expression period can be controlled" include a nucleic acid encoding the nucleic acid altering enzyme complex of the present disclosure under control of an induction regulatory region. Examples of "induction regulatory region" include, but are not particularly limited to, an operon of a temperature sensitive (ts) mutant repressor and an operator controlled thereby. Examples of ts mutant repressors include, but are not limited to, ts mutants of a λ phage derived cI repressor. A λ phage derived cI repressor (ts) binds to an operator and suppresses gene expression downstream at 30°C or less (e.g., 28°C), but dissociates from an operator at a high temperature of 37°C or higher (e.g., 42°C) so that gene expression is induced. Hence, a host cell introduced with a nucleic acid encoding a nucleic acid altering enzyme complex is generally cultured at 30°C or less, the temperature is raised to 37°C or higher at a suitable time, the cell is cultured for a certain period of time and subjected to homologous recombination to introduce a mutation into a target gene. After this process, the temperature can be quickly returned to 30°C or less to minimize the period during which expression of the target gene is suppressed. Even if a gene that is essential for a host cell is targeted, the gene can be edited efficiently while suppressing side effects.

When a temperature sensitive mutation is utilized, for example, insertion of a temperature sensitive mutant of a protein that is required for autonomous replication of a vector into a vector comprising a DNA encoding the nucleic acid altering enzyme complex of the present disclosure results in autonomous replication being disabled quickly after expression of the nucleic acid altering enzyme complex, and the vector naturally falling off with cell division. Examples of such a temperature sensitive mutant protein include, but are not limited to, a temperature sensitive mutant of Rep101 ori which is required for replication of pSC101 ori. At 30°C or less (e.g., 28°C), Rep101 ori (ts) acts on pSC101 ori to enable autonomous replication of a plasmid, but loses the function at 37°C or higher (e.g., 42°C), so that the plasmid would no longer be able to autonomously replicate. Thus, transient expression of the nucleic acid altering enzyme complex of the present disclosure and plasmid removal can be simultaneously performed by concurrent use with a cI repressor (ts) of λ phage described above.

Transient expression of the nucleic acid altering enzyme complex of the present disclosure can be materialized by introducing a DNA encoding the nucleic acid altering enzyme complex into a host cell under the control of an inducible promoter (e.g., lac promoter (induced by IPTG), cspA promoter (induced by cold shock), araBAD promoter (induced by arabinose), or the like), adding an inducer to a medium (or removing the inducer from a medium) at a suitable time to induce expression of the nucleic acid altering enzyme complex, culturing the cell for a certain period of time, and subjecting the cell to a nucleic acid alteration reaction to introduce a mutation into a target gene.

Another aspect of the present disclosure provides an Argonaute-guide RNA complex, comprising: an Argonaute protein; and a synthesized guide RNA polynucleotide, wherein the guide sequence is designed so that the Argonaute-guide RNA complex is directed to a target DNA molecule having a target proximate motif sequence.

Further, another aspect provides an Argonaute-guide RNA complex for use in manipulating a gene function by a site-specific action on a target DNA molecule, the Argonaute-guide RNA complex comprising: an Argonaute protein; and a synthesized guide RNA polynucleotide, wherein the guide sequence is designed so that the Argonaute-guide RNA complex is directed to a target DNA molecule having a target proximate motif sequence, wherein the complex binds to the target molecule to site-specifically act on the target DNA molecule, thereby manipulating the gene function.

Furthermore, another aspect provides an Argonaute-guide RNA complex for use in regulating expression of a target DNA molecule, the Argonaute-guide RNA complex comprising: an Argonaute protein; and a synthesized guide RNA polynucleotide, wherein the guide sequence is designed so that the Argonaute-guide RNA complex is directed to a target DNA molecule having a target proximate motif sequence, wherein the complex binds to the target molecule to regulate expression of the target DNA molecule.

In one embodiment, the Argonaute-guide RNA complex of the present disclosure may be provided in a state where the complex is formed outside of a living body, or the Argonaute-guide RNA complex of the present disclosure can also be provided by operably placing a gene sequence expressing each element of the complex in a vector DNA and forming the complex in a living body from the vector DNA.

Another aspect of the present disclosure provides a system or kit for manipulating a gene function by a site-specific action on a target DNA molecule, the system or kit comprising: two or more complexes comprising an Argonaute protein, a guide RNA, and an added sequence, or two or more thereof; and a means or reagent for contacting the complexes with the target DNA molecule, wherein the guide RNA is designed to guide the complexes to a region comprising a site of interest in the target DNA molecule.

Further, another aspect provides a system or kit for regulating expression of a target DNA molecule, the system or kit comprising: two or more complexes comprising an Argonaute protein, a guide RNA, and an added sequence, or two or more thereof; a means or reagent for dissociating at least a part of a double stranded DNA sequence in the target DNA molecule; and a means or reagent for contacting the complexes with the double stranded DNA sequence at least a part of which has been dissociated, wherein the guide RNA is designed to guide the complexes to a region comprising the double stranded DNA sequence at least a part of which has been dissociated.

In one embodiment of the present disclosure, the kit of the present disclosure can be generally separated into two or more sections to provide each element to be provided (e.g., a vector, a nucleic acid construct, a cell that has been subjected to gene introduction of a nucleic acid of interest, a reagent, a label, a manual, or the like). In one embodiment, the kit of the present disclosure can also be provided in such a manner as to provide each element as a separate kit. In this case, the complex of the present disclosure can be formed in a living body or outside of a living body from a vector DNA or the like designed to be suitably expressed in a cell.

In one embodiment of the present disclosure, the system of the present disclosure is not particularly limited as long as it comprises a means or reagent required for forming the complex of the present disclosure in a living body or outside of a living body, regulating expression of a target DNA molecule, and/or site-specifically acting on the target DNA molecule, thereby enabling manipulation of a gene function.

### (General techniques)

The molecular biological approach, biochemical approach, and microbiological approach used herein are well known and conventional approaches in the art that are described in, for example, Sambrook J. et al. (1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J. J. et al. (1999). PCR Applications: Protocols for Functional Genomics, Academic Press, Bessatsu Jikken Igaku [Experimental Medicine, Supplemental Volume], Idenshi Donyu & Hatsugen Kaiseki Jikken Ho [Experimental Methods for Transgenesis & Expression Analysis], Yodosha, 1997 and the like, the relevant portions (which can be the entire document) of which are incorporated herein by reference.

For DNA synthesis techniques and nucleic acid chemistry for making an artificially synthesized gene, for example, gene synthesis or fragment synthesis service such as GeneArt, GenScript, or Integrated DNA Technologies (IDT) can be used. In addition, said techniques and chemistry are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (1996). Bioconjugate Techniques, Academic Press and the like, the relevant portions of which are incorporated herein by reference.

As used herein, "or" is used when "at least one or more" of the listed matters in the sentence can be employed. When explicitly described herein as "within the range" of "two values", the range also includes the two values themselves.

Reference literatures such as scientific literatures, patents, and patent applications cited herein are incorporated herein by reference to the same extent that the entirety of each document is specifically described.

As described above, the present disclosure has been described while showing preferred embodiments to facilitate understanding. The present disclosure is described hereinafter based on Examples. The aforementioned description and the following Examples are not provided to limit the present disclosure, but for the sole purpose of exemplification. Thus, the scope of the present disclosure is not limited to the embodiments and Examples specifically described herein and is limited only by the scope of claims.

### [Examples]

### (Example 1: Preparation of a recombinant RsAgo protein)

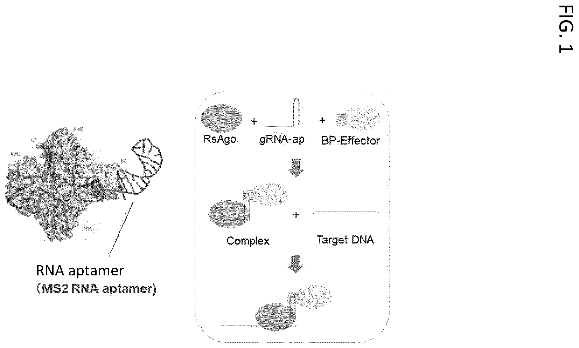

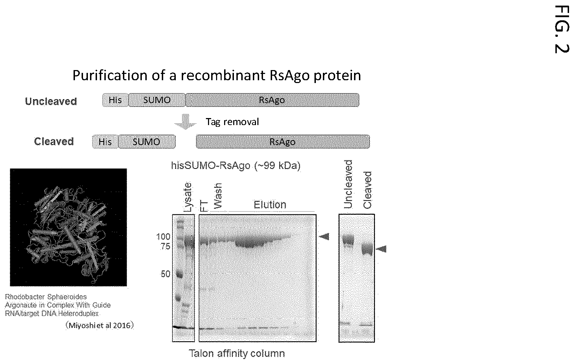

In order to confirm whether an RsAgo complex can recognize a target sequence by fusion of an aptamer to a gRNA as shown in Figure **1****,** a recombinant RsAgo protein was designed and purified according to the following procedure as shown in Figure **2****.**
Plasmid: pT7SU_RsAgo (SEQ ID NO: 1)
Strain: Rosetta2(DE3) pLysS

### (1) Transformation

First, Rosetta2(DE3) pLysS was transformed with pT7SU_RsAgo by a heat shock method, followed by seeding on a Kan50 + Cm34 plate and incubation overnight at 37°C.

### (2) Protein purification

The obtained transformant was cultured overnight at 30°C in LB (Kan + Cm) + 1% glucose (20 ml). 10 ml of o/n culture broth was seeded in 0.9 L of LB (Kan + Cm) + 1% glucose and cultured at 37°C until OD600 1.0 was achieved. IPTG was added thereto so that the final concentration would be 0.5 mM, and the mixture thereof was cultured overnight at 25°C (up to 21 hr) .

### (3) Preparation of a cell extract

Bacterial cells were collected by a centrifugal operation. The bacterial cells were suspended in about 40 ml of Lysis buffer (50 mM Na-phos, pH 8.0; 100 mM NaCl; 10% glycerol; Complete protease inhibitor cocktail (Roche)). DNaseI (final 4 units/ml), MgCl₂ (final 2 mM), and lysozyme (final 2 mg/ml) were added thereto, 20 ml of suspension was mixed with 20 ml of lysis buffer, and the mixture thereof was diluted. The product thereof was frozen with liquid nitrogen, followed by thawing with ice water. The above operation was repeated five times to pulverize the bacterial cells.

Ultracentrifugation was performed for 1 hour at 124 k × g at 4°C and the supernatant was collected. Subsequently, the salt concentration was adjusted to 500 mM and the product thereof was clarified with a 0.45 um filter.

### (4) Protein purification

AKTA purifier was used to perform protein purification under the following condition.
HiTrap TALON_crude 1 ml column
Binding buffer: 50 mM of Na-phos, pH 8.0; 500 mM of NaCl; 5% glycerol
Elution buffer: 50 mM of Na-phos, pH 8.0; 500 mM of NaCl; 5% glycerol, 500 mM of Imidazole
Elution: 30 ml of 2 to 100% B gradient

Fractions were collected based on a UV 280 nm signal, and purified protein was confirmed by SDS-PAGE.

### (5) Tag removal

Purified Ulp1 protease was added to the collected fractions, and the mixture thereof was incubated overnight on ice. Subsequently, tag cleavage was confirmed by SDS-PAGE.

### (6) Protein purification

Imidazole was removed by a desalting column (HiTrap Desalting 5 ml). Subsequently, the product thereof was made pass through a HiTrap TALON crude column again and a flow-through fraction was collected. This fraction was concentrated by ammonium sulfate precipitation and suspended in 8/3 × Storage buffer (54 mM of Tris-Cl, pH 7.5; 1334 mM of NaCl; 14 mM of 2-mercaptoethanol).

The suspension was concentrated by Amicon 15 (MWCO 10k) and dialyzed with 1 × Storage buffer (20 mM of Tris-Cl, pH 7.5; 500 mM of NaCl; 5 mM of 2-mercaptoehanol; 50% glycerol) to prepare a recombinant RsAgo protein.

### (Example 2: EMSA Confirmation of the nucleic acid binding capability)

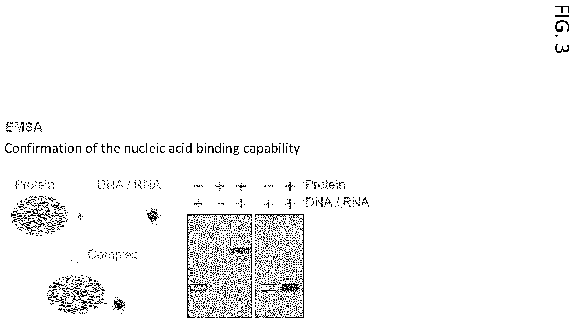

The nucleic acid binding capability of the RsAgo protein obtained in Example 1 was confirmed according to the following procedure (Figure 3).

### (1) Purified protein

A dilution series of purified RsAgo protein was prepared and used.

### (2) Nucleic acid (oligo)

The following nucleic acids were used as a nucleic acid to which the protein was bound.
3'-FAM labelled synthetic RNA (18 nt) 5'-UUACAACCUACUACCUCG-[FAM] (SEQ ID NO: 2)
5'-FAM labelled synthetic DNA (18 nt) 5'-[FAM]-CGAGGTAGTAGGTTGTAA (SEQ ID NO: 3)

### (3) Reaction buffer

20 mM of HEPES-KOH, pH 7.5; 200 mM of NaCl; and 5 mM of MgCl₂ were used as reaction buffer.

### (4) EMSA

The purified protein and synthetic oligonucleotide were mixed in the presence of the above reaction buffer and incubated for 20 minutes at 25°C. After sucrose was added to the mixture thereof, formation of a complex was observed in a 6% TBE polyacrylamide gel. Detection was performed with Blue light LED.

### (5) Phosphorylation of RNA

3'-FAM labelled synthetic RNA was phosphorylated according to the following procedure.

Takara T4 Polynucleotide kinase and ATP were mixed and reacted for 1 hour at 37°C to phosphorylate the mixture. The product thereof was purified by using QIAGEN Nucleotide removal kit.

### (6) Result

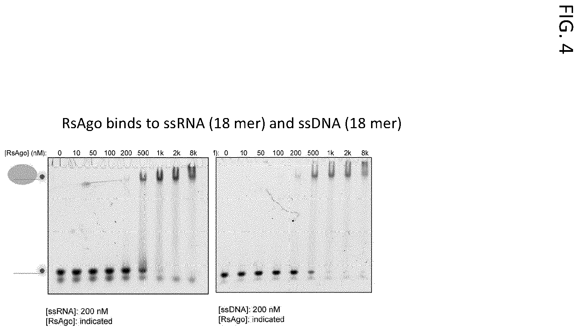

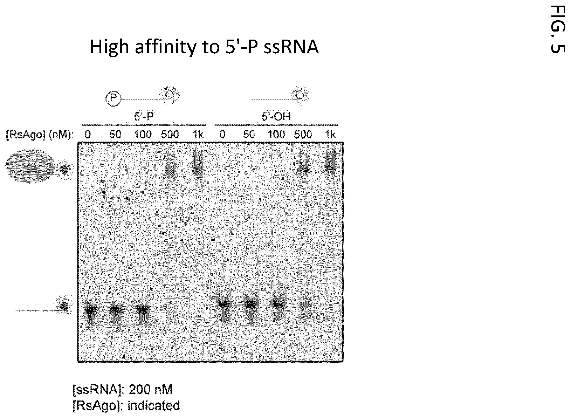

As shown in Figure **4****,** it was found that RsAgo binds to ssRNA (18 mer) and ssDNA (18 mer) . It was also found from the result of Figure **5** that RsAgo has high affinity to 5'-P ssRNA.

### (Example 3: EMSA Confirmation of the nucleic acid binding capability)

Furthermore, in order to confirm the sequence dependency of the RsAgo protein obtained in Example 1 with respect to a target sequence, the nucleic acid binding capability to each of a complementary sequence and a non-complementary sequence was confirmed.

### (1) Purified protein

A dilution series of purified RsAgo protein was prepared and used.

### (2) Nucleic acid (phosphorylated)

The following nucleic acid was used as a phosphorylated nucleic acid.
3'-FAM labelled synthetic RNA (18 nt) 5'-UUACAACCUACUACCUCG-[FAM] (SEQ ID NO: 4)

### (3) Synthetic oligo DNA

The following nucleic acids were used as a nucleic acid to which the protein was bound (SEQ ID NOs: 5 to 8 from the top in order).
* Nucleic acids having a complementary sequence (20, 30, and 40 nt)
* A non-complementary sequence (42 nt)
   20 nt 3'-TCAATGTTGGATGATGGAGC-5'
   30 nt 3'-GAGATCAATGTTGGATGATGGAGCGTACCC-5'
   40 nt 3'-AAGGGGAGATCAATGTTGGATGATGGAGCGTACCCAGTAG-5'
   NC 3'-CGGGTTACCTCATGAAGAACAGGTACCATATAGAGGAAGAAT-5'

### (4) Reaction buffer

20 mM of HEPES-KOH, pH 7.5; 200 mM of NaCl; and 5 mM of MgCl₂ were used as reaction buffer.

### (5) EMSA

The purified protein (final 200 nM) and labelled RNA (final 200 nM) were mixed in the presence of the above reaction buffer and incubated for 15 minutes at 25°C to form a complex. The synthetic oligo DNA was added thereto (final 1 µM or 10 µM). After sucrose was added to the mixture thereof, formation of a complex of the three components was observed in a 6% TBE polyacrylamide gel. Detection was performed with Blue light LED.

### (6) Detection of protein

The acrylamide gel after electrophoresis was stained with CBB to detect protein.

### (7) Result

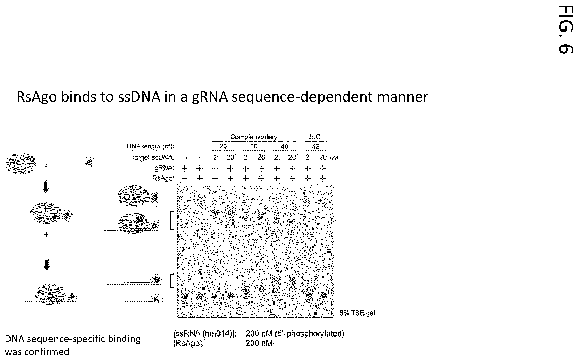

As shown in Figure **6****,** it was found that the RsAgo binds to ssDNA in a gRNA sequence-dependent manner.

### (Example 4: Synthesis of a gRNA-aptamer by in vitro transcription)

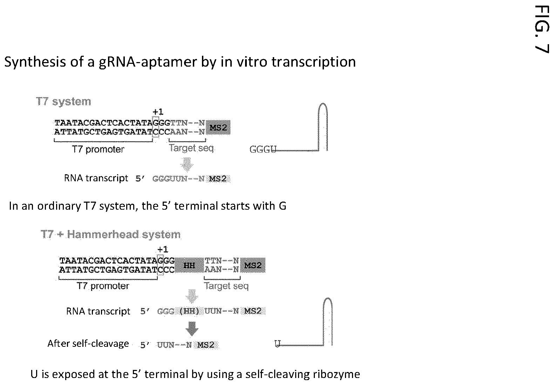

In this example, an aptamer was fused to a guide RNA by in vitro transcription in the manner shown in Figure 7 (the sequences are SEQ ID NOs: 9 to 12 from the top in order in Figure **7**).

### (1) Template plasmid

As a template plasmid, pHMk047_pT7_HH-hm014-MS2 template was used (SEQ ID NO: 16). After digestion with SmaI and linearization, the linear DNA was introduced as a template and reacted for 2 hours at 37°C according to the protocol of Promega Ribomax Large scale RNA production system (T7) kit. The template DNA was digested with DNaseI (for 15 minutes at 37°C) and purified with ZYMO research RNA clean and concentrator kit.

### (2) Hammerhead self-cleavage

The purified RNA and 10 × T4 ligase buffer (NEB) were mixed and incubated for 30 minutes at room temperature (25°C) .

### (3) Phosphorylation

T4 Polynucleotide kinase (NEB) was added, and the mixture thereof was reacted for 30 minutes at 37°C and then for 20 minutes at 65°C to phosphorylate the mixture. The product thereof was purified by using QIAGEN Nucleotide removal kit.

### (4) Result

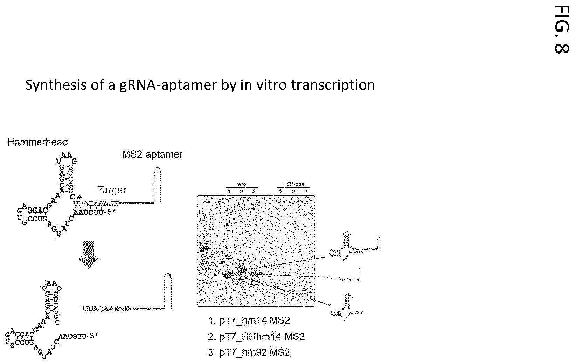

As shown in Figure **8****,** production of a guide RNA added with an aptamer could be observed (the sequences are SEQ ID NOs: 13 to 14 from the top in order in Figure **8**).

### (Example 5: Binding of the gRNA-aptamer to a target sequence)

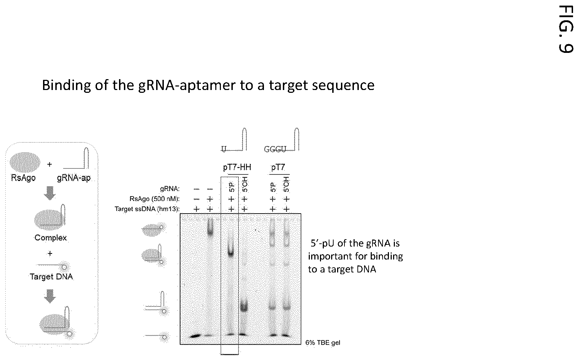

Binding of RsAgo to a target sequence was confirmed by using the gRNA-aptamer obtained in Example 4 (Figure **9**).

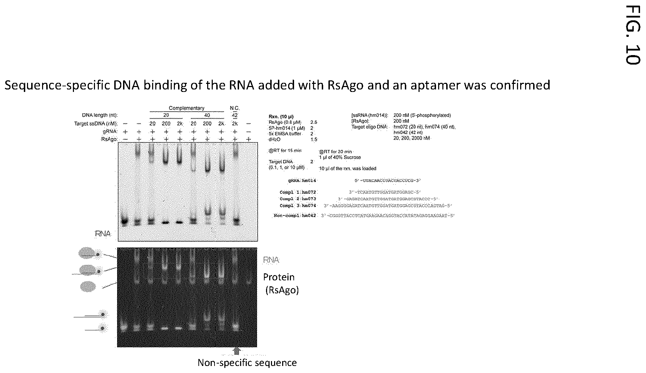

As shown in Figure **10****,** sequence-specific DNA binding of the RNA added with an aptamer and RsAgo could be observed (the sequences are SEQ ID NOs: 5 to 8 from the top in order in Figure **10**).

### (Example 6: EMSA Confirmation of the nucleic acid binding capability)

In this example, a target DNA partially comprising a single strand and a completely double stranded target DNA were prepared and the nucleic acid binding capability was confirmed according to the following procedure in order to confirm whether RsAgo can bind to a double stranded target DNA when binding to a target DNA.

### (1) Purified protein

Purified RsAgo protein was prepared and used.

### (2) Guide RNA (phosphorylated)

The following RNA was used as a phosphorylated guide RNA. 3'-FAM labelled synthetic RNA (18 nt) 5'-UUACAACCUACUACCUCG-[FAM] (SEQ ID NO: 15)

### (3) Target DNA

The following DNAs were used as a target DNA (SEQ ID NOs: 17 to 20 from the top in order).
* DNA partially comprising a single strand
* Completely double stranded DNA

AAGTGGTTCGTGCTTCAA
5′-GGATAACAATTCCCCTCTAG CATGGGTCATCACCATCATC-3′
3′-CCTATTGTTAAGGGGAGATCAATGTTGGATGATCGAGCGTACCCAGTAGTGGTAGTAG-5′

5'-GGATAACAATTCCCCTCTAGTTACAACCTACTACCTCGCATGGGTCATCACCATCATC-3'
3'-CCTATTGTTAAGGGGAGATCAATGTTGGATGATGGAGCGTACCCAGTAGTGGTAGTAG-5'

### (4) Reaction buffer

20 mM of HEPES-KOH, pH 7.5; 200 mM of NaCl; and 5 mM of MgCl₂ were used as reaction buffer.

### (5) EMSA

The purified protein (final 500 nM) and labelled guide RNA (final 100 nM) were mixed in the presence of the above reaction buffer and incubated for 15 minutes at 25°C to form a complex. The target DNA was added (final 100 to 1000 nM) thereto. After sucrose was added to the mixture thereof, formation of a complex of the three components was observed in a 6% TBE polyacrylamide gel.

### (6) Result

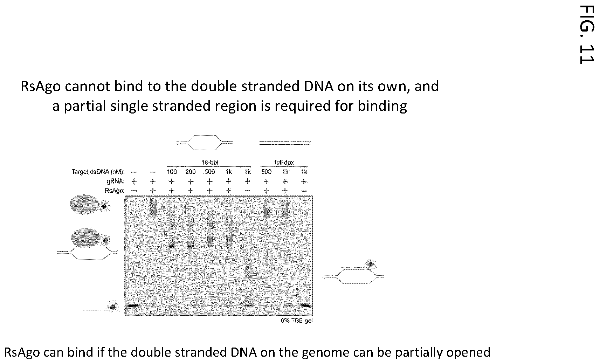

As shown in Figure **11****,** it was found that RsAgo cannot bind to the double stranded DNA on its own, and a partial single stranded region is required for binding.

### (Example 7: EMSA Confirmation of the nucleic acid binding capability)

Next, a target DNA comprising a partial single strand on the 5' side of the target sequence and a target DNA comprising a partial single strand on the 3' side of the target sequence were prepared and the nucleic acid binding capability was confirmed in order to confirm the state of a single stranded region which is required when RsAgo binds to a target DNA.

### (1) Purified protein

Purified RsAgo protein was prepared and used.

### (2) Guide RNA (phosphorylated)

The following RNA was used as a phosphorylated guide RNA. 3'-FAM labelled synthetic RNA (18 nt) 5'-UUACAACCUACUACCUCG-[FAM] (SEQ ID NO: 15)

### (3) Target DNA

The following DNAs were used as a target DNA (SEQ ID NOs: 21 to 24 from the top in order).
* DNA comprising a partial single strand on the 5' side of the target sequence
* DNA comprising a partial single strand on the 3' side of the target sequence

AAGTGGTTCG
5'-GGATAACAATTCCCCTCTAG CTACCTCGCATGGGTCATCACCATCATC-3'
3'-CCTATTGTTAAGGGGAGATCAATGTTGGATGATGGAGCGTACCCAGTAGTGGTAGTAG-5'

                               CGTGCTTCAA
5'-GGATAACAATTCCCCTCTAGTTACAACC          CATGGGTCATCACCATCATC-3'
3'-CCTATTGTTAAGGGGAGATCAATGTTGGATGATGGAGCGTACCCAGTAGTGGTAGTAG-5'

### (4) Reaction buffer

20 mM of HEPES-KOH, pH 7.5; 200 mM of NaCl; and 5 mM of MgCl₂ were used as reaction buffer.

### (5) EMSA

The purified protein (final 500 nM) and labelled guide RNA (final 100 nM) were mixed in the presence of the above reaction buffer and incubated for 15 minutes at 25°C to form a complex. The target DNA was added (final 100 to 1000 nM) thereto. After sucrose was added to the mixture thereof, formation of a complex of the three components was observed in a 6% TBE polyacrylamide gel.

### (6) Result

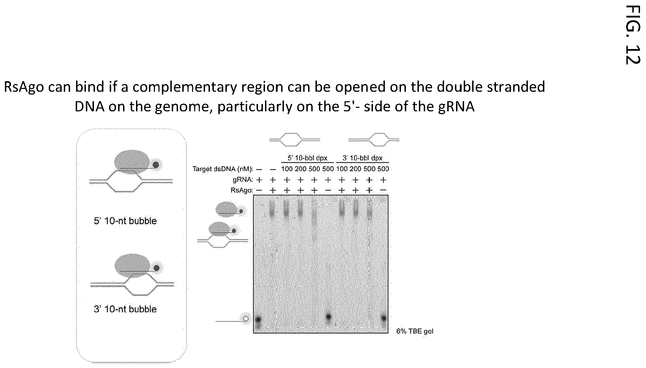

As shown in Figure **12****,** a complex formed in vitro by using: RsAgo purified as a recombinant protein; and a guide RNA added with an MS2 aptamer following any target sequence with a 5' terminal starting with phosphorylated U was found to have a specific binding property to a single stranded DNA or a double stranded DNA partially having a single strand comprising the target sequence. Further, it was found that RsAgo can bind to the target DNA if a complementary region can be opened on the double stranded DNA on the genome, particularly on the 5'- side of the gRNA.

### (Example 8: EMSA Confirmation of the nucleic acid binding capability)

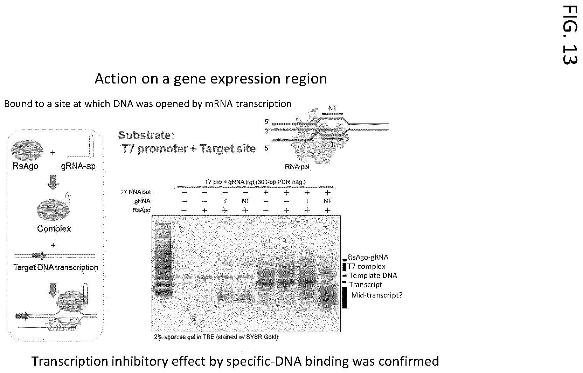

It was found that RsAgo partially requires a single stranded region to bind to a target DNA. Thus, next, it was confirmed whether it would be possible to regulate gene expression by having RsAgo act on a gene expression region (Figure **13**).

### (1) Purified protein

Purified RsAgo protein was prepared and used.

### (2) Guide RNA (phosphorylated)

The following RNAs were used as a phosphorylated guide RNA (SEQ ID NOs: 25 to 26 from the top in order).
3'-FAM labelled in vitro synthetic gRNA aptamer

### Target sequence aptamer

| | |
|---|---|
| T | 5' -TTACAACCTACTACCTCGgggagcACATGAGGATCACCCATGTgcgactcCCACAGTCACTGGGgagtcttcc |
| NT | 5' -CGAGGTAGTAGGTTGTAAgggagcACATGAGGATCACCCATGTgcgactcCCACAGTCACTGGGgagtcttcc |

### (3) Preparation of a target DNA (T7 pol template)

A region comprising a T7 promoter (310 bp) was amplified by PCR with pHMk016 as a template (the template is SEQ ID NO: 27 and the PCR product is SEQ ID NO: 28). The amplification product was purified by FastGene Gel/PCR Extraction Kit (NIPPON Genetics).

### (4) Reaction buffer

80 mM of HEPES-KOH, pH 7.5; 24 of mM MgCl₂; 2 mM of spermidine; and 40 mM of DTT were used as reaction buffer.

### (5) EMSA

The purified protein (final 100 nM) and guide RNA (final 4 µM) were mixed in the presence of the above reaction buffer and incubated for 15 minutes at 25°C to form a complex. The PCR product (target DNA) was added thereto, followed by addition of T7 RNA pol mix (Promega #P1300) and rNTPs (final 1 mM). The mixture thereof was reacted for 20 minutes at 37°C. After sucrose was added to the mixture thereof, the mixture was subjected to electrophoresis with 1.5% agarose gel and stained with SYBR Gold.

### (6) Result

As shown in Figure **13****,** it was confirmed that, when gene expression was induced by using a T7 system in a completely closed double stranded DNA, RsAgo entered and bound to the DNA that was opened as a result of transcription, and further interfered with the gene expression to inhibit normal mRNA synthesis. Especially, the effect was more significant when RsAgo was bound to the strand on the opposite side to the template for transcription.

### (Note)

As described above, the present disclosure is exemplified by the use of its preferred embodiments. It is understood that the scope of the present disclosure should be interpreted solely based on the claims. It is understood that any patent, any patent application, and any references cited herein should be incorporated herein by reference in the same manner as the contents are specifically described herein. The present application claims priority to Japanese Patent Application No. 2020-219833 filed with the JPO on December 29, 2020. The entire content thereof is incorporated herein by reference in the same manner as the contents constitutes the present application in its entirety.

### [Industrial Applicability]

The method of the present disclosure enables artificial manipulation of a gene function in various organisms including animals, plants, and microorganisms. Thus, the method of the present disclosure can be expected to be widely applied to medical application, development of crops, alteration of industrial microorganisms, and the like.

### [Sequence Listing Free Text]

SEQ ID NO: 1: the template plasmid used in Example 1
SEQ ID NOs: 2 to 3: the synthetic RNA or synthetic DNA used in Example 1
SEQ ID NO: 4: the synthetic RNA used in Example 3
SEQ ID NOs: 5 to 8: the synthetic oligo DNAs used in Example 3
SEQ ID NOs: 9 to 12: the synthetic DNAs used in Example 7
SEQ ID NOs: 13 to 14: the guide RNA aptamers used in Example 4
SEQ ID NO: 15: the synthetic RNA used in Example 6
SEQ ID NO: 16: the template plasmid used in Example 4
SEQ ID NOs: 17 to 20: the target DNAs used in Example 6
SEQ ID NOs: 21 to 24: the target DNAs used in Example 7
SEQ ID NOs: 25 to 26: the guide RNA aptamers used in Example 8
SEQ ID NO: 27: the template plasmid used in Example 8
SEQ ID NO: 28: the PCR product of Example 8

## Claims

1. A method for manipulating a gene function by a site-specific action on a target DNA molecule, the method comprising:
providing a complex comprising an Argonaute protein, a guide RNA, and an added sequence; and
contacting the complex with the target DNA molecule,
wherein the guide RNA is designed to guide the complex to a region comprising a site of interest in the target DNA molecule.

2. The method of claim 1, wherein the Argonaute protein has an activity of targeting a DNA.

3. The method of claim 1 or 2, wherein the Argonaute protein has an activity of targeting a DNA at a physiological temperature.

4. The method of any one of claims 1 to 3, wherein the Argonaute protein is derived from a prokaryote.

5. The method of any one of claims 1 to 4, wherein the Argonaute protein is derived from a bacterium.

6. The method of any one of claims 1 to 5, wherein the Argonaute protein is RsAgo derived from *Rhodobacter.*

7. The method of any one of claims 1 to 6, wherein the complex is formed outside of a living body.

8. The method of any one of claims 1 to 7, wherein the complex is formed outside of a living body, and the target DNA molecule is present in a living body.

9. The method of any one of claims 1 to 8, wherein the Argonaute-guide RNA complex is designed to be directed to a target DNA molecule having a target proximate motif sequence.

10. The method of any one of claims 1 to 9, wherein manipulation of the gene function includes gene expression control, gene sequence alteration, and/or both thereof.

11. The method of any one of claims 1 to 9, wherein manipulation of the gene function includes gene expression control.

12. The method of any one of claims 1 to 9, wherein manipulation of the gene function includes gene sequence alteration.

13. A method for regulating expression of a target DNA molecule, the method comprising:
providing a complex comprising an Argonaute protein, a guide RNA, and an added sequence;
dissociating at least a part of a double stranded DNA sequence in the target DNA molecule; and
contacting the complex with the double stranded DNA sequence at least a part of which has been dissociated,
wherein the guide RNA is designed to guide the complex to a region comprising the double stranded DNA sequence at least a part of which has been dissociated.

14. The method of claim 13, wherein the Argonaute protein has an activity of targeting a DNA.

15. The method of claim 13 or 14, wherein the Argonaute protein has an activity of targeting a DNA at a physiological temperature.

16. The method of any one of claims 13 to 15, wherein the Argonaute protein is derived from a prokaryote.

17. The method of any one of claims 13 to 16, wherein the Argonaute protein is derived from a bacterium.

18. The method of any one of claims 13 to 17, wherein the Argonaute protein is RsAgo derived from *Rhodobacter.*

19. The method of any one of claims 13 to 18, wherein the complex is formed outside of a living body.

20. The method of any one of claims 13 to 19, wherein the complex is formed outside of a living body, and the target DNA molecule is present in a living body.

21. An Argonaute-guide RNA complex, comprising:
an Argonaute protein; and
a synthesized guide RNA polynucleotide,
wherein the guide sequence is designed so that the Argonaute-guide RNA complex is directed to a target DNA molecule having a target proximate motif sequence.

22. The complex of claim 21, wherein the Argonaute protein has an activity of targeting a DNA.

23. The complex of claim 21 or 22, wherein the Argonaute protein has an activity of targeting a DNA at a physiological temperature.

24. The complex of any one of claims 21 to 23, wherein the Argonaute protein is derived from a prokaryote.

25. The complex of any one of claims 21 to 24, wherein the Argonaute protein is derived from a bacterium.

26. The complex of any one of claims 21 to 25, wherein the Argonaute protein is RsAgo derived from *Rhodobacter.*

27. An Argonaute-guide RNA complex for use in manipulating a gene function by a site-specific action on a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to site-specifically act on the target DNA molecule, thereby manipulating the gene function.

28. The complex of claim 27, wherein the Argonaute protein has an activity of targeting a DNA.

29. The complex of claim 27 or 28, wherein the Argonaute protein has an activity of targeting a DNA at a physiological temperature.

30. The complex of any one of claims 27 to 29, wherein the Argonaute protein is derived from a prokaryote.

31. The complex of any one of claims 27 to 30, wherein the Argonaute protein is derived from a bacterium.

32. The complex of any one of claims 27 to 31, wherein the Argonaute protein is RsAgo derived from *Rhodobacter.*

33. The complex of any one of claims 27 to 32, wherein manipulation of the gene function includes gene expression control, gene sequence alteration, and/or both thereof.

34. The complex of any one of claims 27 to 32, wherein manipulation of the gene function includes gene expression control.

35. The complex of any one of claims 27 to 32, wherein manipulation of the gene function includes gene sequence alteration.

36. An Argonaute-guide RNA complex for regulating expression of a target DNA molecule, the Argonaute-guide RNA complex comprising:
an Argonaute protein; and
a synthesized guide RNA polynucleotide,
wherein the guide sequence is designed so that the Argonaute-guide RNA complex is directed to a target DNA molecule having a target proximate motif sequence, wherein the complex binds to the target molecule to regulate expression of the target DNA molecule.

37. The complex of claim 36, wherein the Argonaute protein has an activity of targeting a DNA.

38. The complex of claim 36 or 37, wherein the Argonaute protein has an activity of targeting a DNA at a physiological temperature.

39. The complex of any one of claims 36 to 38, wherein the Argonaute protein is derived from a prokaryote.

40. The complex of any one of claims 36 to 39, wherein the Argonaute protein is derived from a bacterium.

41. The complex of any one of claims 36 to 40, wherein the Argonaute protein is RsAgo derived from *Rhodobacter.*

42. A system or kit for manipulating a gene function by a site-specific action on a target DNA molecule, the system or kit comprising:
two or more complexes comprising an Argonaute protein, a guide RNA, and an added sequence, or two or more thereof; and
a means or reagent for contacting the complexes with the target DNA molecule,
wherein the guide RNA is designed to guide the complexes to a region comprising a site of interest in the target DNA molecule.

43. The system or kit of claim 42, wherein the Argonaute protein has an activity of targeting a DNA.

44. The system or kit of claim 42 or 43, wherein the Argonaute protein has an activity of targeting a DNA at a physiological temperature.

45. The system or kit of any one of claims 42 to 44, wherein the Argonaute protein is derived from a prokaryote.

46. The system or kit of any one of claims 42 to 45, wherein the Argonaute protein is derived from a bacterium.

47. The system or kit of any one of claims 42 to 46, wherein the Argonaute protein is RsAgo derived from *Rhodobacter.*

48. The system or kit of any one of claims 42 to 47, comprising a means for forming the complexes outside of a living body.

49. The system or kit of any one of claims 42 to 48, comprising a means for forming the complexes outside of a living body, wherein the target DNA molecule is present in a living body.

50. The system or kit of any one of claims 42 to 49, wherein the Argonaute-guide RNA complexes are designed to be directed to a target DNA molecule having a target proximate motif sequence.

51. The system or kit of any one of claims 42 to 50, wherein manipulation of the gene function includes gene expression control, gene sequence alteration, and/or both thereof.

52. The system or kit of any one of claims 42 to 50, wherein manipulation of the gene function includes gene expression control.

53. The system or kit of any one of claims 42 to 50, wherein manipulation of the gene function includes gene sequence alteration.

54. A system or kit for regulating expression of a target DNA molecule, the system or kit comprising:
two or more complexes comprising an Argonaute protein, a guide RNA, and an added sequence, or two or more thereof;
a means or reagent for dissociating at least a part of a double stranded DNA sequence in the target DNA molecule; and
a means or reagent for contacting the complexes with the double stranded DNA sequence at least a part of which has been dissociated,
wherein the guide RNA is designed to guide the complexes to a region comprising the double stranded DNA sequence at least a part of which has been dissociated.

55. The system or kit of claim 54, wherein the Argonaute protein has an activity of targeting a DNA.

56. The system or kit of claim 54 or 55, wherein the Argonaute protein has an activity of targeting a DNA at a physiological temperature.

57. The system or kit of any one of claims 54 to 56, wherein the Argonaute protein is derived from a prokaryote.

58. The system or kit of any one of claims 54 to 57, wherein the Argonaute protein is derived from a bacterium.

59. The system or kit of any one of claims 54 to 58, wherein the Argonaute protein is RsAgo derived from *Rhodobacter.*

60. The system or kit of any one of claims 54 to 59, further comprising a means for forming the complexes outside of a living body.

61. The system or kit of any one of claims 54 to 60, further comprising a means for forming the complexes outside of a living body, wherein the target DNA molecule is present in a living body.
